(19) Europäisches Patentamt
European Patent Office
Office européen des brevets

(11) Veröffentlichungsnummer : **0 012 710**
**B1**

(12) # EUROPÄISCHE PATENTSCHRIFT

(45) Veröffentlichungstag der Patentschrift : **03.02.82**

(21) Anmeldenummer : **79730012.6**

(22) Anmeldetag : **06.12.79**

(51) Int. Cl.³ : **C 12 P 17/04**, C 07 D307/935, C 12 P 31/00// C12R1/78, C12R1/85, C12R1/645, C12R1/86

(54) **Mikrobiologische Reduktion von 15-Keto-prostaglandin-Zwischenprodukten.**

(30) Priorität : **08.12.78 DE 2853637**

(43) Veröffentlichungstag der Anmeldung : **25.06.80 (Patentblatt 80/13)**

(45) Bekanntmachung des Hinweises auf die Patenterteilung : **03.02.82 Patentblatt 82/05**

(84) Benannte Vertragsstaaten : **AT BE CH DE FR GB IT LU NL SE**

(56) Entgegenhaltungen :
**DE - A - 2 401 671**
**US - A - 3 687 811**

(73) Patentinhaber : **SCHERING AKTIENGESELLSCHAFT**
**Berlin und Bergkamen**
**Müllerstrasse 170/178 Postfach 65 0311**
**D-1000 Berlin 65 (DE)**

(72) Erfinder : **Kieslich, Klaus, Dr.**
**Strasse zum Löwen 24**
**D-1000 Berlin 39 (DE)**
Erfinder : **Radüchel, Bernd, Dr.**
**Gollanczstrasse 132**
**D-1000 Berlin 28 (DE)**
Erfinder : **Skuballa, Werner, Dr.**
**Olwenstrasse 25**
**D-1000 Berlin 28 (DE)**
Erfinder : **Vorbrüggen, Helmut, Prof.**
**Wilkestrasse 7**
**D-1000 Berlin 27 (DE)**
Erfinder : **Dahl, Helmut, Dr.**
**Gollanczstrasse 102**
**D-1000 Berlin 28 (DE)**

**EP 0 012 710 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Mikrobiologische Reduktion von 15-Keto-prostaglandin-Zwischenprodukten

Beschreibung :

Die Erfindung betrifft ein mikrobiologisches Verfahren zur stereoselektiven Reduktion der 15-Ketofunktion in biccyclischen Prostaglandin-Zwischenprodukten zu den entsprechenden 15-Hydroxyverbindungen und zwar derart, daß die 15-Hydroxygruppe ebenso wie die bereits vorhandene 11-Hydroxygruppe α-ständig ist.

Die vorliegende Erfindung eignet sich besonders zur mikrobiologischen Reduktion der 3-Oxo-Gruppe in (1S,5R,6R,7R)-6-[(E)-3-oxo-phenoxy-1-alkenyl]-7-hydroxy-2-oxa-bicyclo [3.3.0] octan-3-onen zur (R)-3-Hydroxygruppe. Von den genannten 3-Oxo-Verbindungen ist die 3-Oxo-4-phenoxy-1-butenyl-Verbindung für die erfindungsgemäße Reduktion besonders bevorzugt. Unter den für die vorgenannte mikrobiologische Reduktion gefundenen Stämmen Kloeckera magna (ATCC 20 109), Kloeckera jensenii (ATCC 20 110), Saccharomyces carlsbergensis (CBS 1506), Saccharomyces carlsbergensis (CBS 1513) und Hansenula anomala (NRRL-Y-366) hat sich besonders der Stamm Kloeckera jensenii (ATCC 20 110) bewährt.

Während die chemische Reduktion der 15-Ketogruppen in Prostaglandinen und Prostaglandin-Zwischenprodukten z.B. mit Natriumborhydrid nur über Gemische der entsprechenden 15α- und 15β-Hydroxyverbindungen und wegen der sich anschließenden Trennung nur unter Ausbeuteverlusten zu den gewünschten 15α-Hydroxyprostaglandinen führt, sind durch die US-PS 3 687 811 eine Reihe von Mikroorganismen bekannt, die die 15-Ketogruppe in 11-Hydroxy-15-oxo-prostaglandinen je nach bereits vorhandener Konformation der 11-Hydroxygruppe in die entsprechende trans-15-Hydroxygruppe umwandeln. Mit dem Verfahren aus der DE-OS 23 57 815 verfügt der Stand der Technik über eine mikrobiologische Methode, die z.B. ein 11-Hydroxy-15-oxo-prostaglandin in ein Gemisch aus 11α,15α- und 11β,15β-Dihydroxy-prostaglandin umwandelt. Die cis-Anordnung der 11α,15α-Hydroxygruppen entspricht der jenigen in biologisch aktiven Prostaglandinen.

Alle genannten Verfahren versagen jedoch bei der mikrobiologischen Reduktion von (1S,5R,6R,7R)-6-[(E)-3-oxo-phenoxy-1-alkenyl]-7-hydroxy-2-oxa-bicyclo [3.3.0]-octan-3-onen. Auch das in der DE-OS 24 01 761 beschriebene mikrobiologische Verfahren arbeitet nur mit sehr geringen Ausbeuten. Es hat außerdem den Nacheeil, daß die für die Reduktion verwendeten Basidiomyceten als höhere Pilzarten nur ein langsames Wachstum besitzen und sich schlechter handhaben lassen.

Es wurde nun gefunden, daß sich auch (1S,5R,6R,7R)-6-[(E)-3-oxo-phenoxy-1-alkenyl]-7-hydroxy-2-oxa-bicyclo [3.3.0]-octan-3-one in 3-Position der Seitenketter mit sehr guten Ausbeuten zu entsprechenden 3-Hydroxyverbindungen reduzieren lassen, wenn man Kloeckera-, Saccharomyces- oder Hansenula-Stämme verwendet. Das erfindungsgemäße Verfahren läßt sich mit Vorteil auch auf (1S,5R,6R,7R)-6-[(E)-3-oxo-1-alkenyl]-7-hydroxy-2-oxa-bicyclo-[3.3.0] octan-3-one anwenden.

Die Erfindung betrifft somit ein Verfahren zur stereoselektiven Reduktion deer 15-Ketogruppe in bicyclischen Prostaglandin-Zwischenprodukten zu 15α-Hydroxyverbindungen der allgemeinen Formel I

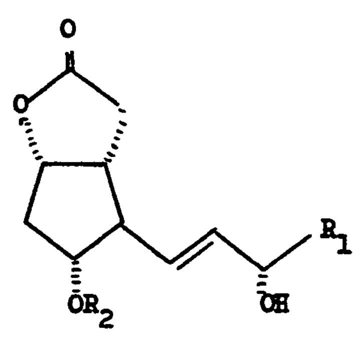

(I)

worin

$R_1$ einen Phenoxymethylrest, der gegebenenfalls im Phenyl durch Halogen oder Trifluormethyl substituiert sein kann, oder einen geradkettigen oder verzweigten Alkylrest mit 1-5 C-Atomen und

$R_2$ Wasserstoff, Acetyl, Benzoyl oder p-Phenylbenzoyl bedeuten, dadurch gekennzeichnet, daß man ein Keton der allgemeinen Formel II

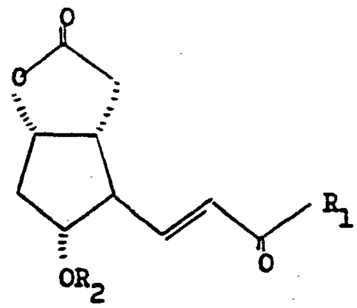

(II)

worin

$R_1$ und $R_2$ die obenangegebenen Bedeutungen haben, mit Kloeckera-, Saccharomyces- oder Hansenula-Stämmen behandelt und das dabei entstehende 15-Hydroxy-Prostaglandin-Zwischenprodukt isoliert.

Unter Halogen werden Fluor, Chlor und Brom verstanden, wobei Fluor und Chlor bevorzugt sein sollen.

Wenn der Phenylring im Phenoxymethylrest (aus der Definition für $R_1$) durch Halogen oder Trifluormethyl substituiert ist, kommt dafür die 3- oder 4-Stellung in Betracht. Bevorzugte Stellung ist die in 3. Folgende Reste für einen substituierten Phenoxymethylrest kommen in Frage : 3-Chlorphenoxymethyl, 4-Chlorphenoxymethyl, 3-Fluorphenoxymethyl, 4-Fluorphenoxymethyl, 3-Trifluormethylphenoxymethyl, 4-Trifluormethylphenoxymethyl. Unter geradkettigen oder verzweigten Alkylresten mit 1-5 C-Atomen sind die Reste Methyl, Äthyl, n-Propyl, Isopropyl, n-Butyl, sek.-Butyl, Isobutyl, tert.-Butyl, n-Pentyl, Isopentyl, Neopentyl, sek.-Pentyl, tert.-Pentyl. Bevorzugt sind die geradkettigen Reste mit 3-5 C-Atomen.

Besonders vorteilhaft arbeitet das Verfahren, wenn $R_2$ in Verbindungen der Formel II Benzoyl oder p-Phenylbenzoyl bedeutet.

Je nach der letztlich gewünschten Bedeutung von $R_2$ können die Schutzgruppen Benzoyl oder p-Phenylbenzoyl nach an sich bekannten Methoden abgespalten werden. Aus den nach dem erfindungsgemäße Verfahren hergestellten Verbindungen der Formel I lassen sich unter Erhaltung des Asymmetriezentrums in 15-Position (entsprechend Prostaglandin-Nomenklatur) pharmakologisch wirksame Prostaglandine herstellen. Vom (1S,5R,6R,7R)-6-[(E)-3-oxo-4-phenoxy-1-butenyl]-7-benzoyloxy-2-oxa-bicyclo [3.3.0] octan-3-on ausgehend gelangt man in einer mehrstufigen Synthese zum Wirkstoff Sulproston (beschrieben in DE-OS 23 55 540) :

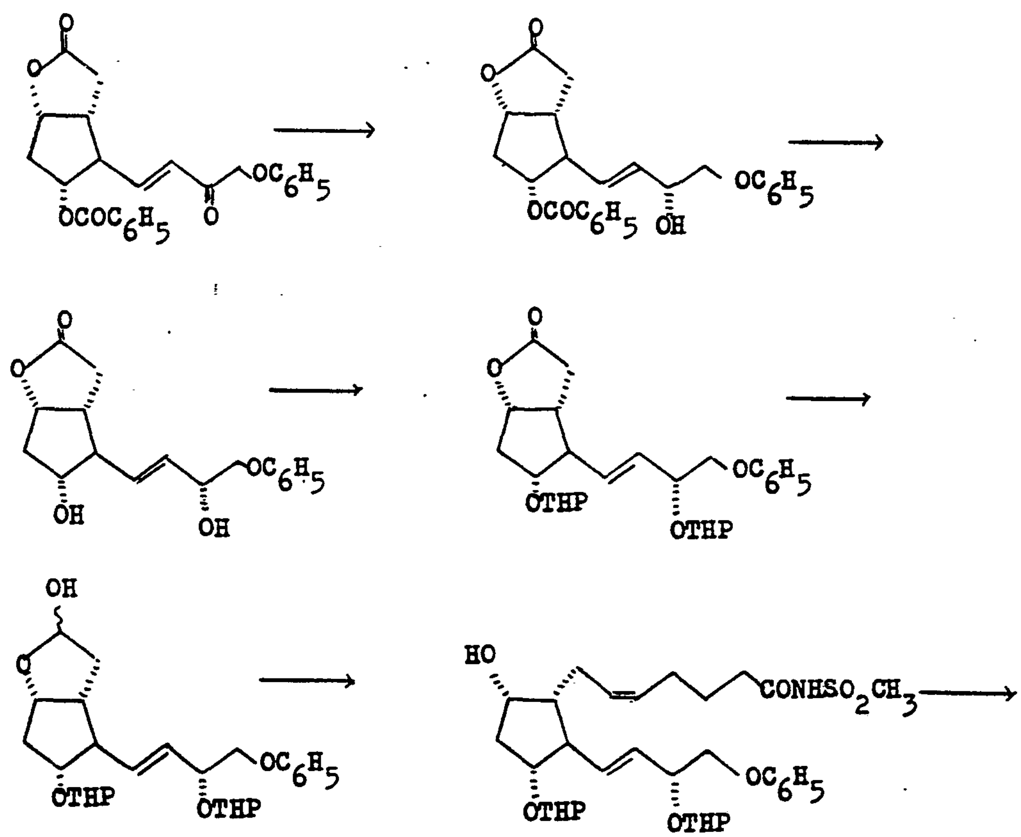

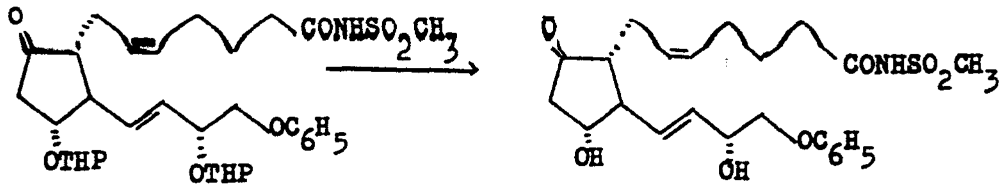

Unter den verschiedenen Arten der angegebenen Klassen von Mikroorganismen treten naturgemäß Unterschiede der Wirksamkeit in der erfindungsgemäßen Reduktion auf. Von Kloeckera magna (ATCC 20 109), Kloeckera jensenii (ATCC 20 110), Saccharomyces carlsbergensis (CBS 1506), Saccharomyces carlsbergensis (CBS 1513) und von Hansenula anomala (NRRL-Y-366) liegen gute Ergebnisse vor, wobei Reduktionen mit dem Stamm Kloeckera jensenii (ATCC 20 110) besonders gute Ausbeuten ergeben.

Zunächst werden unter den für die genannten Mikroorganismen üblicherweise verwendeten Kulturbedingungen in einem geeigneten Nährmedium und unter Belüften Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd. Die Emulgierung des Substrats kann beispielsweise bewirkt werden, in dem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin(R), Tagat(R), Tween(R) und Span(R) beispielsmäßig genannt.

Oft ermöglicht die Emulgierung der Substrate einen erhöhten Substratsdurchsatz und somit eine Steigerung der Substratskonzentration. Es ist aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Umwandlungen allgemein erforderlich ist, im Einzelfall durch Vorversuche, wie sie dem Fachmann geläufig sind, ermittelt werden.

## Beispiel 1

In Erlenmeyerkolben mit 21 Fassungsvermögen werden je 500 ml einer Nährlösung aus 5,0 % Glucose, 2,0 % Cornsteep liquor, beschickt und bei 120° 30 Minuten im Autoklaven sterilisiert. Die Kolben werden mit einer Abschwemmung von einer 3-tätigen Schrägagarkultur von Kloeckera magna (ATCC 20109) beimpft und 48 Stunden auf einer Rotationsschüttelmaschine mit einer Frequenz von 145 U/Min. bei 30° geschüttelt. Je 100 ml dieser Vorkultur dienen zur Beimpfung gleichartig vorbereiteter Kolben, in die nach einer Schüttelzeit von 6 Stunden Lösungen von je 125 mg (1S,5R,6R,7R)-6-[(E)-3-oxo-4-phenoxy-1-butenyl]-7-benzoyloxy-2-oxa-bicyclo-[3.3.0]-octan-3-on in 5 ml DMSO zugesetzt werden. Durch Entnahme von Intervallproben mit Dünnschichtanalytik wird die Abnahme des Ausgangsmaterials verfolgt. Bei weitgehend vollständiger Umwandlung wird dreimal mit je 200 ml Methylisobutylketon extrahiert ; die vereinigten Extrakte werden im Vakuum eingeengt ; der Rückstand wird durch Säulenchromatographie an Silicagel mit einer Gradientenelution Hexan-Methylenchlorid aufgetrennt. Das aus den Mittelfraktionen erhaltene (1S,5R,6R,7R,3'R)-6-[(E)-3-hydroxy-4-phenoxy-1-butenyl]-7-benzoyloxy-2-oxa-bicyclo-[3.3.0]-octan-3-on wird aus Methylenchlorid/Hexan-Gemisch umkristallisiert.

Fp. 129/133-134°

## Beispiel 2

Unter den bei Beispiel 1 angegebenen Bedingungen werden zur gewünschten Ketoreduktion verwendet :

Saccharomyces carlsbergensis CBS 1513.

## Beispiel 3

Saccharomyces carlsbergensis CBS 1506.

## Beispiel 4

Hansenula anomala (NRRL-Y-366).

Beispiel 5

Kloeckera jensenii (ATCC 20110).

Beispiel 6

In einem Erlenmeyerkolben mit 2 l Fassungsvermögen werden 500 ml einer Nährlösung aus 5,0 % Glucose und 2,0 % Cornsteep-liquor beschickt und bei 120° 30 Minuten im Autoklaven sterilisiert. Der Kolben wird mit einer Abschwemmung von einer 3-tägigen Schrägagarkultur von Kloeckera jensenii (ATCC 20110) beimpft und 48 Stunden auf einer Rotationsschüttelmaschine mit einer Frequenz von 145 U/Min. bei 30° geschüttelt. Mit 250 ml dieser Vorkultur wird ein 20 l Fermenter, der 15 l eines sterilisierten Mediums gleicher Zusammensetzung enthält, beimpft und nach Zugabe einiger Tropfen Antischaummittel (Polypropylen 2000) bei 29° unter Belüftung 1 Vol Luft/Vol Fermenterbrühe/Min. und Ruhren mit 220 U/Min. fermentiert. Nach einer Laufzeit von 24 Stunden werden 900 ml der so erhaltenen Kultur in einen gleichartig angesetzten Fermenter steril überführt und unter gleichen Bedingungen fermentiert. Nach 12 h werden 10 g Tween 80 und nach 14 h eine Lösung von 3,75 g (1S,5R,6R,7R)-6-[(E)-3-oxo-4-phenoxy-1-butenyl]-7-benzoyloxy-2-oxa-bicyclo-[3.3.0]-octan-3-on in 150 ml DMSO zugesetzt. Nach 12 Stunden weiterer Fermentationszeit wird die Kulturbrühe 3 mal mit je 5 l Methylisobutylketon extrahiert. Die vereinigten Extrakte werden in einem Vakuumumlaufverdampfer bei maximal 40° eingeent. Das Rohprodukt dieses Ansatzes wird an Silicagel chromatographiert und mit einem Gradientensystem Hexan/Methylenchlorid (1 : 1) ansteigend bis Methylenchlorid eluiert. Nach einem Vorlauf enthalten die folgenden Fraktionen das gewünschte (1S,5R,6R,7R,3'R)-6-[(E)-3-hydroxy-4-phenoxy-1-butenyl]-7-benzoyloxy-2-oxa-bicyclo-[3.3.0]-octan-3-on, was aus Tetrachlorkohlenstoff umkristallisiert 2,25 g (60 % d.Th.) Reinprodukt ergibt.

Fp. 129/135-136°.

Beispiel 7

Unter gleichen Bedingungen wie Beispiel 6 wurde mit Hansenola anomola (NRRL-Y-366) fermentiert. Man erhielt (1S,5R,6R,7R,3'R)-6-[(E)-3-hydroxy-4-phenoxy-1-butenyl]-7-benzoyloxy-2-oxa-bicyclo [3.3.0]-octan-3-on vom Schmelzpunkt 120/121-122°.

Beispiel 8

Unter den Bedingungen des Beispiel 1 wurden mit einer Kultur von Kloeckera magna (ATCC 20109) 125 mg (1S,5R,6R,7R)-6-((E)-3-oxo-1-octenyl)-7-p-phenylbenzoyloxy-2-oxabicyclo [3.3.0]-octan-3-on in 68 Stunden Kontaktzeit fermentiert und aufgearbeitet. Durch chromatographische Reinigung des Rohproduktes erhielt man ein Öl, identisch mit dem authentischen Material von E. Corey et al. J. Chem. Soc. *93,* 1491 (1971).

**Ansprüche**

1. Verfahren zur Herstellung von 15α-Hydroxy-Prostaglandin-Zwischenprodukten der allgemeinen Formel I

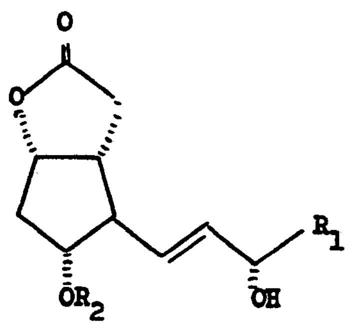

(I)

worin

$R_1$ einen Phenoxymethylrest, der gegebenenfalls im Phenyl durch Halogen oder Trifluormethyl substituiert sein kann, oder einen geradkettigen oder verzweigten Alkylrest mit 1-5 C-Atomen und

$R_2$ Wasserstoff, Acetyl, Benzoyl oder p-Phenylbenzoyl bedeuten, dadurch gekennzeichnet, daß man ein 15-Keton der allgemeinen Formel II

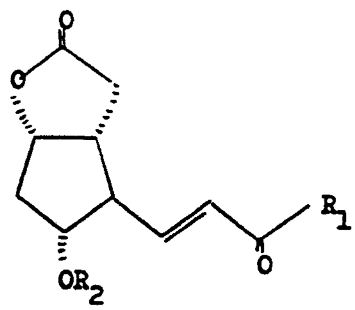

(II)

worin

$R_1$ und $R_2$ die obenangegebenen Bedeutungen haben, mit Kloeckera-, Saccharomyces- oder Hansenula-Stämmen behandelt und das dabei entstehende 15α-Hydroxy-Prostaglandin-Zwischenprodukt isoliert.

2. Verfahren nach Anspruch 1, dadurch gekennzeichnet, daß man ein 15-Keton der Formel II mit Kloeckera jensenii (ATCC 20110) behandelt und das dabei entstehende 15α-Hydroxy-Prostaglandin-Zwischenprodukt isoliert.

3. Verfahren nach Anspruch 2, dadurch gekennzeichnet, daß man als Ausgangsprodukt (1S,5R,6R,7R)-6-[(E)-3-oxo-phenoxy-1-alkenyl]-7-hydroxy-2-oxa-bicyclo [3.3.0] octan-3-one einsetzt.

4. Verfahren nach Anspruch 2, dadurch gekennzeichnet, daß man als Ausgangsprodukt (1S,5R,6R,7R)-6-[(E)-3-oxo-4-phenoxy-1-butenyl]-7-hydroxy-2-oxa-bicyclo [3.3.0] octan-3-on einsetzt.

**Claims**

1. Process for the manufacture of 15α-hydroxyprostaglandin intermediates of the general formula I

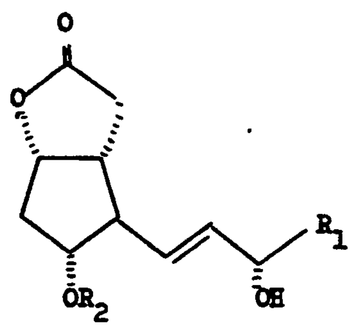

(I)

in which

$R_1$ represents a phenoxymethyl radical that can optionally be substituted in the phenyl moiety by halogen or trifluoromethyl, or a straight-chain or branched alkyl radical having from 1 to 5 carbon atoms, and

$R_2$ represents hydrogen, acetyl, benzoyl or p-phenylbenzoyl, characterised in that a 15-ketone of the general formula II

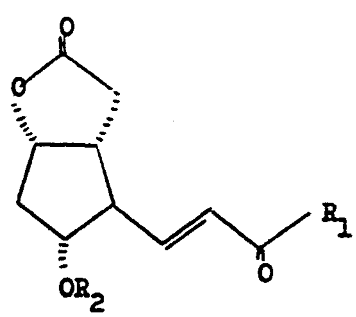

(II)

in which

$R_1$ and $R_2$ have the meanings given above, is treated with Kloeckera, Saccharomyces or Hansenula strains and the resulting 15α-hydroxyprostaglandin intermediate is isolated.

2. Process according to claim 1, characterised in that a 15-ketone of the formula II is treated with Kloeckera jensenii (ATCC 20110) and the resulting 15α-hydroxyprostaglandin intermediate is isolated.

3. Process according to claim 2, characterised in that (1S,5R,6R,7R)-6-[(E)-3-oxo-phenoxy-1-alkenyl]-7-hydroxy-2-oxa-bicyclo [3.3.0] octan-3-ones are used as starting material.

4. Process according to claim 2, characterised in that (1S,5R,6R,7R)-6-[(E)-3-oxo-4-phenoxy-1-butenyl]-7-hydroxy-2-oxa-bicyclo [3.3.0] octan-3-one is used as starting material.

**Revendications**

1. Procédé de préparation de composés intermédiaires à groupe hydroxy en 15α pour prostaglandines, composés qui répondent à la formule générale I

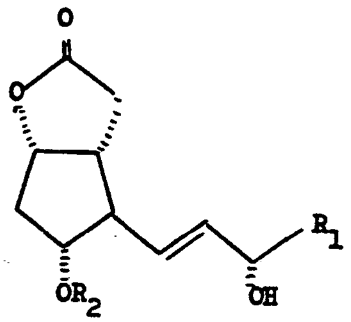

(I)

dans laquelle

$R_1$ représente un radical phénoxyméthyle éventuellement porteur, sur son radical phényle, d'un halogène ou d'un groupe trifluorométhyle, ou un radical alkyle en $C_1$-$C_5$, linéaire ou ramifié, et

$R_2$ représente l'hydrogène ou un radical acétyle, benzoyle ou p-phénylbenzoyle, procédé caractérisé en ce qu'on traite une cétone en 15 répondant à la formule générale II

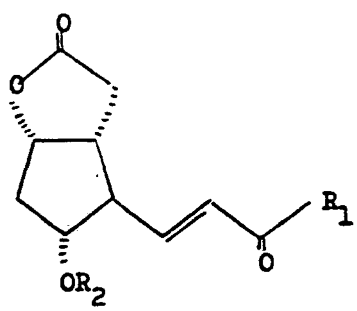

(II)

dans laquelle

$R_1$ et $R_2$ ont les significations précédemment données, par des souches de Kloeckera, de Saccharomyces ou d'Hansenula et on isole le composé intermédiaire à groupe hydroxy en 15α pour prostaglandines qui s'est ainsi formé.

2. Procédé selon la revendication 1, caractérisé en ce qu'on traite une cétone en 15 de formule II par Kloeckera jensenii (ATCC 20110) et on isole le composé intermédiaire hydroxylé en 15α pour prostaglandines qui s'est ainsi formé.

3. Procédé selon la revendication 2, caractérisé en ce qu'on utilise, comme corps de départ, des [oxo-3 phénoxy-alcène-1 yl-(E)]-6 hydroxy-7 oxa-2 bicyclo [3.3.0] octanones-3 (1S,5R,6R,7R).

4. Procédé selon la revendication 2, caractérisé en ce qu'on utilise, comme corps de départ, l'[oxo-3 phénoxy-4 butène-1 yl-(E)]-6 hydroxy-7 oxa-2 bicyclo [3.3.0] octanone-3 (1S,5R,6R,7R).